# EUROPEAN PATENT APPLICATION

(11) **EP 2 821 092 A1**
(43) Date of publication of application: **07.01.2015**
(21) Application number: 14167206.3
(22) Date of filing: 22.12.2005
(51) Int. Cl.: A61M 16/06, A62B 7/00, A61H 31/00, A62B 9/02

(54) **Respiratory mask having gas washout vent and gas washout vent assembly for respiratory mask**

(30) Priority: 30.12.2004 US 640184 P
(62) Divisional of application: 05818610.7
(71) Applicant: ResMed Ltd., Bella Vista, New South Wales 2153 (AU)
(72) Inventor: Henry, Robert Edward, Bella Vista New South Wales 2153 (AU); Drew, Johanne Elizabeth, Bella Vista New South Wales 2153 (AU); Crumblin, Geoffrey, Bella Vista New South Wales 2153 (AU); Virr, Alexander, Bella Vista New South Wales 2153 (AU)
(74) Representative: Vossius & Partner

(57) **Abstract**

A vent assembly for use with a respiratory mask of the type used in CPAP treatment. In one embodiment, the vent is made of a thin air permeable membrane (28). The membrane can be made of a hydrophobic material such as expanded polytetrafluoroethylene (PTFE). An expanded PTFE membrane is mounted on a polypropylene scrim. The pores of the ePTFE membrane have a reference pore size of 10 to 15 microns. Alternatively, the vent assembly includes a stainless steel vent having holes with diameters less than about 0.2 mm. In another embodiment, the membrane has a superficial cross-sectional area of approximately 500 mm2. In further embodiments, a vent of a mesh material, e.g., an auxetic vent (200) or a PTFE mesh, may be used as an air permeable membrane, either alone or in combination with a traditional vent structure.

## Description

### CROSS REFERENCE TO PRIORITY APPLICATION

This application claims the benefit of U.S. Provisional Application No. 60/640,184, filed December 30, 2004, incorporated herein by reference in its entirety.

### CROSS-REFERENCES TO APPLICATIONS

U.S. Patent Application No. 10/976,874, filed November 1, 2004, pending, which is a continuation of U.S. Patent Application No. 10/377,110, filed March 3, 2003, now U.S. Patent No. 6,823,865, which is a continuation of U.S. Patent Application No. 09/570,907, filed on May 15, 2000, now U.S. Patent No. 6,581,594, are incorporated herein by reference in their entireties.

### BACKGROUND OF THE INVENTION

The present invention relates to a respiratory mask and a vent for a respiratory mask.

The application of Continuous Positive Airway Pressure (CPAP) via a nasal mask is a common ameliorative treatment for sleep disordered breathing (SDB) including obstructive sleep apnea (OSA) as described in commonly-assigned U.S. Patent No. 4,944,310, incorporated herein by reference in its entirety. In CPAP treatment for OSA, air or other breathable gas is supplied to the entrance of a patient's airways at a pressure elevated above atmospheric pressure, typically in the range 3-20 cm H₂O, as measured in the patient interface. It is also known for the level of treatment pressure to vary during a period of treatment in accordance with patient need, that form of CPAP being known as automatically adjusting nasal CPAP treatment, as described in commonly-assigned U.S. Patent No. 5,245,995, incorporated herein by reference in its entirety.

Non-invasive positive pressure ventilation (NIPPV) is another form of treatment for breathing disorders including sleep disordered breathing. In a basic form NIPPV involves a relatively high pressure of gas being provided in the patient interface during the inspiratory phase of respiration and a relatively low pressure or atmospheric pressure being provided in the patient interface during the expiratory phase of respiration. In other NIPPV modes the pressure can be made to vary in a complex manner throughout the respiratory cycle. For example, the pressure at the patient interface during inspiration or expiration can be varied through the period of treatment as disclosed in the commonly-assigned International PCT Patent Application No. WO 98/12965 and International PCT Patent Application No. WO 99/61088, both incorporated herein by reference in their entireties.

In this specification any reference to CPAP treatment is to be understood as embracing all of the above-described forms of ventilatory treatment or assistance.

Typically, the patient interface for CPAP treatment includes a nasal mask. The nasal mask is generally defined by a mask shell which forms an inner cavity defined by its interior surface, mask cushion and the user's face, a gas inlet which may or may not include a separate component such as a swivel elbow. Alternatively, a nose-mouth mask or full-face mask or nasal prongs or nasal pillows can be used. In this specification any reference to a mask is to be understood as incorporating a reference to a nasal mask, nose-mouth mask, full face mask, nasal prongs or nasal pillows unless otherwise specifically indicated. The mask incorporates, or has in close proximity, a gas washout vent for venting exhaled gases to atmosphere. The gas washout vent (the vent) is sometimes referred to as a CO₂ washout vent.

It is important that the apparatus is quiet and comfortable to encourage patient compliance with therapy. The exhausting to atmosphere of exhaled gas through the vent creates noise. As CPAP and NIPPV treatments are normally administered while the patient is sleeping, minimization of such noise is desirable for both the comfort of the patient and any bed partner. It is also desirable to minimize the exhaust gas jet by diffusing the air flow, in order to avoid any further disturbance or noise which may be caused by the exhaust gas jet hitting bedding or other obstacles.

From a clinical perspective it is desirable for a mask and vent combination to maximize both the elimination of exhaled CO₂ through the vent and also the inhalation of the supplied breathable gas. In this way, retention of exhaled CO₂ within the mask, which is "rebreathed' by the wearer, is minimized. Generally by locating the vent in the mask shell, CO₂ washout will be superior to locating the same vent between the mask shell and the breathable gas supply conduit.

It is desirable to minimize the weight of the vent assembly for greater patient comfort.

Systems for the delivery of nasal CPAP treatment often incorporate in-line humidifiers to minimize drying of the nasal mucosa and increase patient comfort. Accordingly, it is also desirable that a vent not block when used with humidified gas. It is also desirable that a vent be easily cleaned or economically disposable.

A number of vent configurations are known. One approach to vent configuration is to create within the mask shell one or more openings that allow for the flow of exhaust gas from the inner cavity to atmosphere. The exhaust flow may be directed through the incorporation of an additional pipe extending out from the opening located on the mask shell outer surface.

The Assignee's nasal mask system known by the name "ResMed Modular Mask System" incorporates an outlet vent located in the swivel elbow connected to the mask shell. The ports defining the vent have the same cross-sectional thickness and are formed from the same polycarbonate material that is used to form the swivel elbow and mask shell frame.

The whisper swivel, manufactured by Respironics, Inc., provides three slots on the circumference of a generally cylindrical attachment piece. In use, the attachment piece is to be interposed between the mask shell and the gas conduit. The attachment piece is made of the same material and thickness as is used to make the mask shell.

European Patent No. 0 697 225, which is incorporated herein by reference in its entirety, discloses a vent formed from a porous sintered material.

A known vent, manufactured by Gottleib Weinmann Gerate Fur Medizin Und Arbeitsschutz GmbH and Co., comprises a generally cylindrical insert to be interposed, in use, between the mask shell and the gas conduit. The insert includes a window which is covered with a porous sintered material of approximately 3-4 mm thickness.

Another type of vent intended to be inserted between the mask shell and the breathable gas supply conduit is the E-Vent N by Draeger Medizintechnik GmbH (the Draeger vent). The Draeger vent comprises a stack of 21 annular disks that have slots in their adjacent surfaces for gas to flow therethrough. Each slot has a length of 5 to 7 mm, as measured along the path from the interior of the vent to atmosphere.

The Assignee produces a respiratory mask known as the MIRAGE® nasal mask system and the MIRAGE® full-face mask (the MIRAGE® mask). The MIRAGE® mask has a crescent shaped opening in the mask shell in which is located a complementary shaped crescent elastometric-insert with six holes therein which constitutes the vent. The elastomeric inset has a cross-sectional thickness of 3 to 4 mm. The vent of the type used in the MIRAGE® is described in International Patent Application No. WO 98/34665 and Australian Patent No. 712236, both of which are incorporated herein in their entireties.

It is an aspect of the present invention to provide an alternative form of vent that is suitable for use in a respiratory mask.

### BRIEF SUMMARY OF THE INVENTION

One aspect of the present invention provides a vent assembly suitable for use with a mask used in CPAP treatment wherein the vent assembly is a thin air permeable membrane.

In one form of the invention, the membrane is thinner than the mask frame.

In another form of the invention, the membrane is thinner than 0.5 mm.

In another form of the invention the membrane has an approximate thickness of 0.05 mm.

In another form of the invention the membrane is constructed from a hydrophobic material such as polytetrafluoroethylene (PTFE).

In embodiments, the membrane may comprise a mesh material, such as a porous fabric or a PTFE mesh. The mesh material may be capable of thickening upon stretching, e.g., by use of an auxetic material including auxetic fibers. The fibers may have negative Poisson's Ratio, as well as low Young's Modulus (to enable easy stretching). The auxetic material can be in the form of sheet material having one or more perforations and/or slits, instead of a mesh.

In another form of the invention the membrane is constructed from expanded PTFE.

In another form of the invention the expanded PTFE membrane is mounted on a polypropylene scrim.

In another form, the pores of the membrane have a reference pore size of 10 to 15 microns.

In another form of the invention the membrane is constructed from stainless steel. The stainless steel sheet has a thickness of approximately 0.45 mm and a number of holes, each hole having a diameter of approximately 0.1 mm. The total open area of such a stainless steel membrane is approximately 5%.

In another form of the invention the membrane of the vent has a superficial cross-sectional area of approximately 500 mm².

In another form of the invention the vent assembly comprises a membrane attached to a vent frame, the vent assembly forming an insert which can be remove ably attached to a mask frame.

In another form of the invention there is provided a respiratory mask for communicating breathable gas to the entrance of a wearer's airways, the mask including (i) mask shell, (ii) a gas inlet and (iii) an opening into which an insert constructed from a thin air permeable membrane with a corresponding shape may be placed. The opening may be positioned in the mask shell or in the gas inlet.

In one form, the mask includes a mask shell with an integrally formed gas inlet and the opening is provided in the mask shell remote the inlet. In another form, the mask includes a mask shell with an integrally formed gas inlet and the opening is provided in the gas inlet. In yet another form, the mask includes a mask shell with a separately formed gas inlet attached thereto and the opening is provided in the mask shell remote the inlet. In still yet another form, the mask includes a mask shell with a separately formed gas inlet attached thereto and the opening is provided in the gas inlet.

Another aspect of the present invention provides a respiratory mask arrangement for communicating breathable gas to the entrance of a wearer's airways, the mask arrangement including a vent assembly comprising an opening with a thin air permeable membrane extending across an opening.

In further aspects, the present invention provides an apparatus for delivering CPAP, which apparatus includes a mask arrangement for communicating breathable gas to the entrance of a wearer's airways, the mask arrangement including a gas washout vent assembly comprising an opening with a thin air permeable membrane extending across said opening.

These and other aspects will be described in or apparent from the following detailed description of preferred embodiments.
The following aspects are preferred embodiments of the present invention.
1. A respiratory mask comprising:
   a patient interface having a cushion that at least in part defines a breathing cavity; and
   a gas washout vent comprising a thin air permeable membrane formed, constructed and arranged to allow gas to exit from the breathing cavity,
   wherein the membrane comprises a mesh material.
2. A respiratory mask according to aspect 1, wherein the mesh material comprises a polytetrafluoroethylene mesh having a pore size of about 105µm, an open area of about 32% and a thickness of about 155 µm.
3. The respiratory mask of any one of aspects 1-2, wherein the gas washout vent further comprises an air restrictive element disposed beneath the mesh material.
4. The respiratory mask of aspect 3, wherein the air restrictive element is disposed beneath the mesh material with essentially no gap between the air restrictive element and the mesh material.
5. The respiratory mask of any one of aspects 3-4, wherein the air restrictive element is disposed beneath the mesh material with a gap of about 0.5 - 1mm between the air restrictive element and the mesh material.
6. The respiratory mask of aspect 1, wherein the mesh material includes one or more auxetic fibers configured to expand upon application of a stretching force.
7. The respiratory mask of aspect 6, wherein the auxetic fibers have a relatively low Young's Modulus in the range of about 0.1 to 10.
8. The respiratory mask of any one of aspects 6-7, wherein the auxetic fibers have a negative Poisson's Ratio.
9. The respiratory mask of any one of aspects 1 or 6-8, wherein the mesh material includes fibers having a diameter configured to increase with increases in pressure supplied to the breathing chamber.
10. The respiratory mask of any one of aspects 1 or 6-9, wherein the mesh material is configured to bulge with increased pressure.
11. The respiratory mask of any one of aspects 1 or 6-10, wherein a flow rate of gas through the vent is designed to remain substantially constant with changes in pressure in the cavity.
12. The respiratory mask of any one of aspects 1-11, wherein an open area of the vent is configured to decrease with increased pressure in the cavity.
13. A respiratory mask comprising:
   a patient interface having a cushion that at least in part defines a breathing cavity; and
   a gas washout vent including an air permeable member to allow gas to exit from the breathing cavity, wherein the member has a surface area, a thickness, at least one of pores and holes with a length and diameter, and a total open area due to the presence of the pores and holes that are selected to help eliminate or reduce noise while maintaining sufficient CO₂ washout during patient breathing, wherein the thickness of the member is less than 3mm.
14. The respiratory mask according to aspect 13, wherein the thickness is about 0.5mm.
15. The respiratory mask according to aspect 13, wherein the thickness is 0.45mm.
16. The respiratory mask according to aspect 13, wherein the thickness is 0.05mm.
17. A respiratory mask comprising:
   a mask shell that can be fitted over a user s nose;
   a cushion positioned in proximity to an edge portion of the mask shell to aid in fitting the mask shell to a user's face;
   a breathable gas inlet that can conduct gas through the mask shell to a breathing cavity formed between the mask shell and a user's face when the mask is in use; and
   a gas washout vent comprising a vent structure configured to cause a pressure drop in a stream of breathable gas as the breathable gas flows through the gas washout vent and a hydrophobic mesh membrane disposed outwardly of the vent structure.
18. The respiratory mask of aspect 17, wherein the hydrophobic mesh membrane comprises a polytetrafluoroethylene mesh membrane.
19. The respiratory mask of aspect 18, wherein the polytetrafluoroethylene mesh membrane has a pore size of about 105µm, an open area of about 32% and a thickness of about 155µm.
20. The respiratory mask of any one of aspects 17-19, wherein the hydrophobic mesh membrane is disposed outwardly of the vent structure with essentially no gap between the vent structure and the hydrophobic mesh membrane.
21. The respiratory mask of any one of aspects 17-20, wherein the hydrophobic mesh membrane is disposed outwardly of the vent with a gap of about 0.5 - 1mm between the vent structure and the hydrophobic mesh membrane.
22. A mask comprising:
   a patient interface with a cushion defining at least in part a breathing cavity;
   a vent provided to exhaust gas washout from the breathing cavity, the vent including a thin air permeable membrane having a plurality of holes having a diameter of about 0.1mm, an open area of about 5% in a region containing the holes, and a total area of about 300-400mm².
23. The mask of aspect 22, wherein the total area is about 320-330mm².
24. The mask of any one of aspects 22-23, wherein the holes are tapered along an axial length thereof.
25. The mask of any one of aspects 22-24, wherein the diameter of an exterior of the mask is marginally smaller than at the interior of the mask.
26. A mask comprising:
   a patient interface including a cushion that defines at least in part a breathing cavity; and
   a gas washout vent made at least in part from an auxetic material.
27. The mask of aspect 26, wherein the auxetic material includes one or more slits and/or perforations.
28. The mask of any one of aspects 26-27, wherein the vent includes a sail portion made of substantially non-porous material, the auxetic material being provided between the sail portion and a frame portion of the mask.
29. The mask of any one of aspects 26-28 wherein a flow rate of gas through the vent is designed to remain substantially constant with changes in pressure in the cavity.
30. The mask of any one of aspects 26-29 wherein an open area of the vent is configured to decrease with increased pressure in the cavity. 6e

### DESCRIPTION OF THE DRAWINGS

The present invention will be described with reference to the following drawings, in which like numerals represent like structures throughout the several views, and in which:
Fig. 1 is a perspective view of a respiratory mask according to a first embodiment of the invention;
Fig. 2 is a perspective view of a respiratory mask according to a second embodiment of the invention;
Fig. 3 is a perspective view of a respiratory mask according to a third embodiment of the invention;
Fig. 4 is a partial cross-sectional view of a vent assembly according to a fourth embodiment of the invention;
Fig. 5 is a partial cross-sectional view of a vent assembly according to a fifth embodiment the invention;
Fig. 6 is a perspective view of a respiratory mask according to sixth embodiment of the invention;
Fig. 7 is a perspective view of a full-face mask according to a seventh embodiment of the invention;
Fig. 8 is an enlarged detailed view of an insert suitable for use with the masks shown in Fig. 6 and Fig 7;
Fig. 9 is a perspective view of a vent assembly according to an eighth embodiment of the invention where the thin air permeable membrane is located in a cylindrical position on a tube suitable for attachment to the mask elbow;
Figs. 10-16 describe alternative embodiments of the present invention in which auxetic fibers are used in conjunction with the vent assembly;
Fig. 17 illustrates a vent according to yet another embodiment of the present invention; and
Fig. 18 is a mask with a vent according to still another embodiment of the present invention.

### DETAILED DESCRIPTION OF PREFERRED EMBODIMENTS

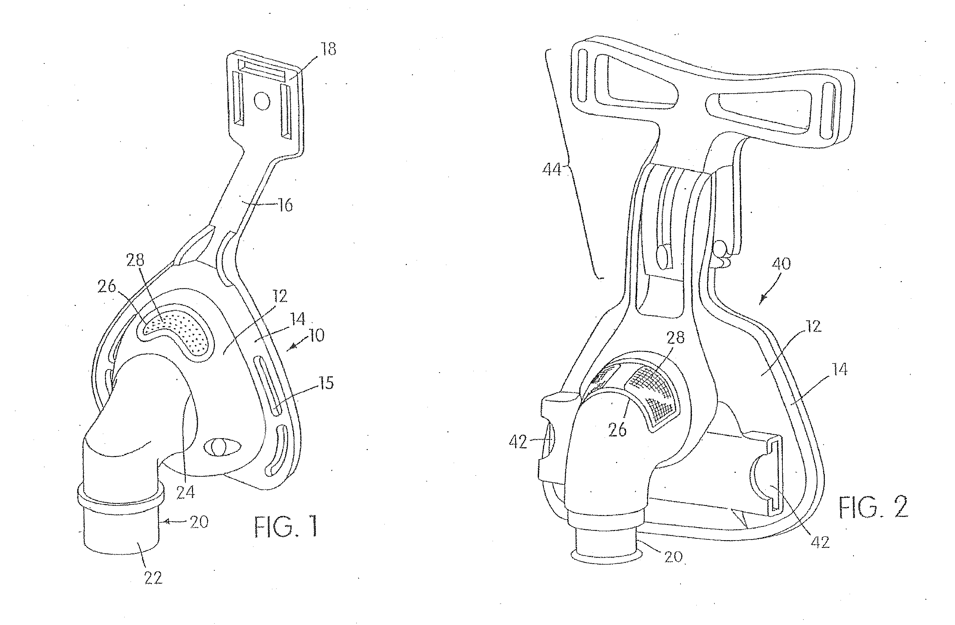

Fig. 1 shows a patient interface, e.g., a nasal respiratory mask 10, according to a first embodiment of the invention. The mask 10 includes a rigid plastic mask shell 12 that has a peripheral flange 14 for mounting of a cushion (not shown) to the shell 12. The cushion abuts the wearer's face in use and is well known in the art. The flange 14 includes slots 15 for the connection of mask restraining straps (not shown) that extend around the head of the wearer to maintain the mask 10 adjacent to the wearer's face. The straps are also known in the art. The shell 12 also includes an arm 16, which terminates in a fitting 18 that is adapted to connect to a forehead support (not shown), which is also known in the art.

The mask shell 12 includes a breathable gas inlet 20 which is rotatably mounted to the shell 12. The inlet 20 has a first end 22 which is adapted for connection with a breathable gas supply conduit (not shown) and a second end 24 which is adapted to connect to, and communicate the supplied gas to the interior of the shell 12 for subsequent communication with the wearer's airways.

The mask 10 includes a gas washout vent constituted by an opening 26 in the shell 12 across which extends a thin air permeable membrane 28.

In the Fig 1 embodiment, the thin air permeable membrane 28 is a stainless steel sheet approximately 0.45 mm thick having holes with a diameter approximately 0.1 mm in diameter. The total open area is approximately 5% of the total superficial surface area of the sheet. The dimensions of the sheet are approximately 322 mm². The holes are, e.g., laser cut into the stainless steel. The holes are desirably laser cut or flame cut through the stainless steel, and in general, have a diameter less than about 0.2 mm.

Preferably the holes have a diameter of less than 0.2 mm, and preferably provide a total open area of approximately 1% to 25% of the superficial surface area of the steel. The holes may be tapered (in a gradual or stepped manner) through their internal bore. In use, if the larger end of the vent's openings are located on the atmosphere side of the vent, the opportunity for blockage occurring by the insertion of particulate matter will be minimized, because larger particles may not be able to enter the smaller end of the vent's openings on the inside of the vent. Alternatively, the smaller end of the vent's openings may be located on the atmosphere side, which may make the vent quieter.

In another example, the membrane includes a plurality of holes each having a diameter in the range of about 0.1-0.3mm, with an open area ranging from 5 to 60%, and sufficient area (i.e., number of holes) to provide adequate flow at low pressures such as 4cmH₂O to meet the requirements to flush CO₂. A preferred embodiment of this invention is a mask shell that has a thickness of 0.45mm, holes with a diameter of 0.1mm that are drilled, molded, laser cut or otherwise constructed therethrough, an open area of about 5% in the region containing the holes, and a total area of the vent of about 300-400mm², preferably about 320-330mm², and more preferably about 322mm². Preferably, the holes are tapered such that the diameter at the exterior of the mask is marginally smaller than that at the interior of the mask.

Fig. 2 shows a nasal respiratory mask 40 according to a second embodiment of the invention. Like reference numerals to those used in describing the first embodiment will be used to denote like features in respect of the second embodiment. Accordingly, the mask 40 has a shell 12 with a gas inlet 20. Instead of the slots 15 of the first embodiment the mask shell includes openings 42 which are adapted to snap engage with connection fittings (not shown) provided on the end of mask restraining straps (not shown). Further, instead of the arm 16 and fitting 18, the mask 40 includes an adjustable forehead support mechanism indicated generally by the reference numeral 44.

The mask 40 also includes a vent constituted by an opening 26 formed in the gas inlet 20 across which extends a thin air permeable membrane 28.

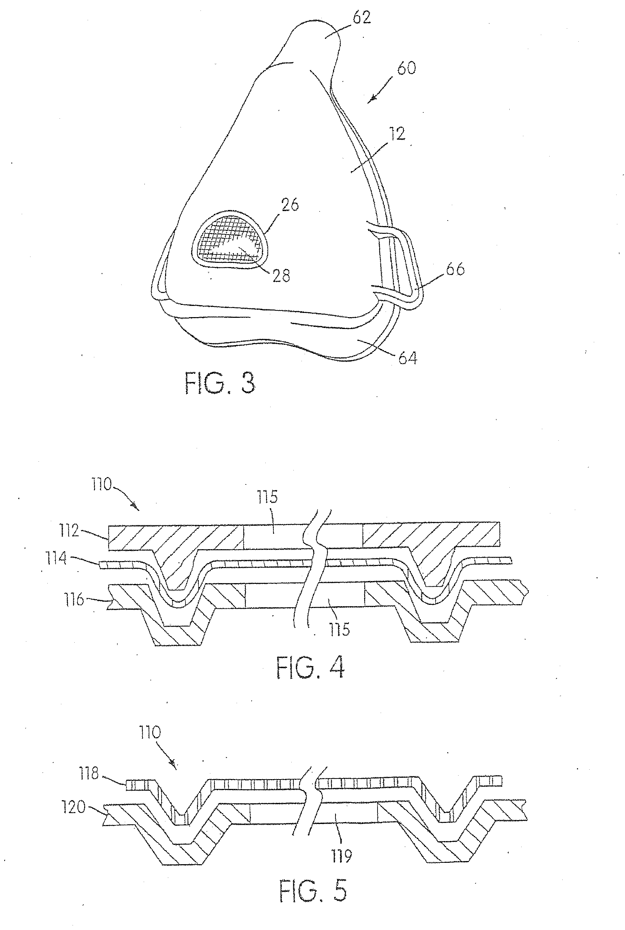

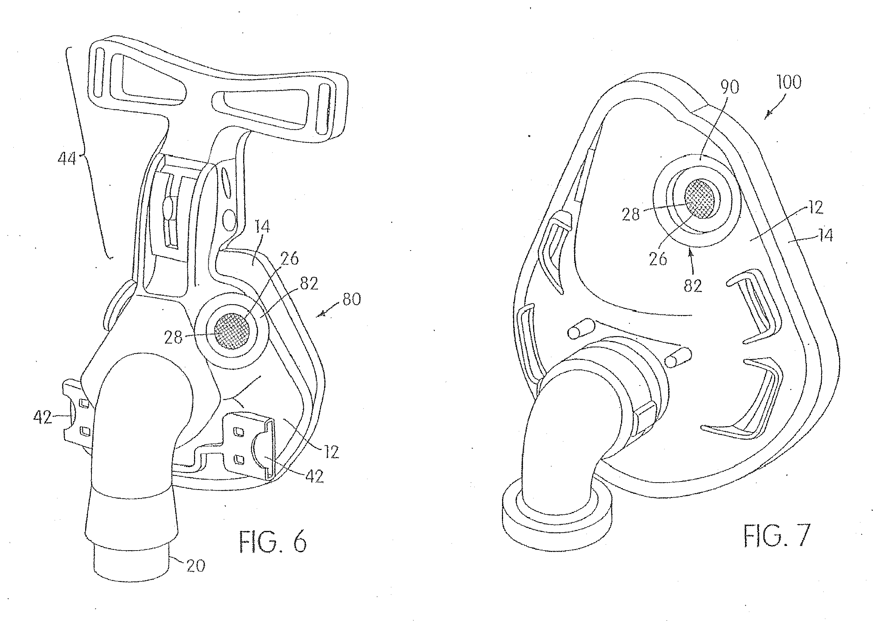

Fig. 3 shows a mask 60 according to a third embodiment of the present invention. Although this particular embodiment is directed to a nasal mask, it should noted that various vent arrangements can be used with various mask arrangements. Once again like reference numerals to those used in describing features of the first embodiment shall be used to denote like features in respect of the third embodiment. The mask 60 includes a mask shell 12 with an integrally formed fixed gas inlet 62. A cushion 64 is attached to the peripheral flange 14 (Fig. 2) of the shell 12. The shell 12 also includes slotted extensions 66 for connecting headgear (not shown) to the mask. The mask 60 includes an opening 26 across which is extended a thin air permeable membrane 28 of identical construction to the ePTFE membrane discussed bellow in relation to the mask 40 shown in Fig. 6.

Fig. 4 shows a cross-section of vent assembly 110. There is provided a membrane 114 interposed between an outer element 112 and an inner element 116. This arrangement provides for a simple assembly. There is a corresponding opening 115 in the outer element 112 and inner element 116 to allow for the passage of air through the membrane. The inner element 116 may form part of the mask frame or of a separate insert to be positioned in an opening in the mask frame.

Fig. 5 shows an alternative cross-section of a vent assembly 110. There is provided a stainless steel membrane insert 118 positioned over the inner element 120. There is an opening 119 in the inner element 120 to allow for the passage of air through the membrane. The inner element 119 may form part of the mask frame or of a separate insert to be positioned in an opening in the mask frame.

Fig. 6 shows a nasal respiratory mask 80 according to a sixth embodiment of the invention. The mask 80 is similar to the second embodiment of the mask 40 shown in Fig. 2 and like reference numerals have been used to indicate like features with respect to the second embodiment. In the mask 40 of Fig. 2, the vent is provided in the gas inlet 20, whereas in the mask 80 the vent is provided in the shell 12. More particularly, the mask 80 includes two cylindrical inserts 82 which have an inner opening 26 across which extends the thin air permeable material 28. The thin air permeable material is made from GORE-TEX® product attached to a polypropylene scrim having an area of 481 mm. The membrane is constructed from expanded polytetrafluoroethylene (ePTFE). The inventors have identified GORE-TEX® ePTFE product manufactured by W. L. Gore & Associates, Inc. of Maryland USA (GORE-TEX® membrane) as being a suitable material for constructing a membrane. In one preferred form, the GORE-TEX® membrane has the following characteristics:
- Membrane material: 100% expanded polytetrafluoroethylene
- Reference pore size: 10-15 micron
- Bubble Point: typical minimum individual 0.02 bar
- Airflow: 0.37 LPM/cm²
- Thickness: 0.05 mm
- Substrate: polypropylene scrim

Fig. 7 shows a seventh embodiment of a full-face respiratory mask 100 according to the invention. Once again like reference numerals to those used in denoting like features with previous embodiments have been used to denote like features in respect of this embodiment. The mask 100 is similar to the mask 80 shown in Fig. 6 in that the vent is provided in the inserts 82. However the mask 100 uses slotted extensions 66 to attach mask restraining straps (not shown), not openings 42.

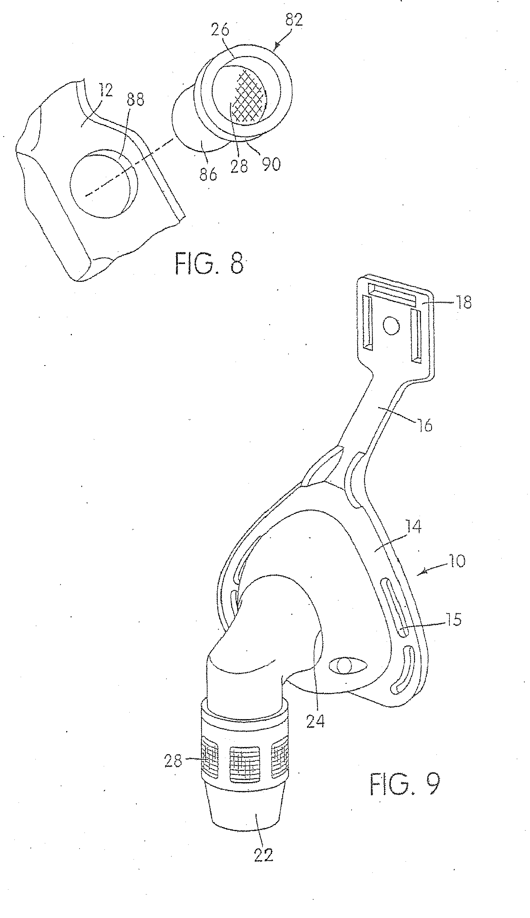

As best seen in Fig. 8, which is a close-up view of the insert shown in Fig. 6, the insert 82 is comprises a cylindrical portion 86 sized to be a snug fit into a circular orifice 88 provided in the mask shell 12. The insert 82 located against the outer surface of the shell 12 by a peripheral flange 90. The inserts may be glued in position.

Fig. 9 shows a further embodiment of the invention in which an in-line vent assembly is provided. Like numerals are used to indicate like features with previous embodiments. In this embodiment, the in-line vent assembly comprises a generally cylindrically shaped vent frame with "windows" or "ports" covered with a membrane as described above.

The thin air permeable membrane of the present invention may be attached to the mask by any suitable means. For example the stainless steel vent described above may be attached to a polycarbonate mask shell by way of hot glue adhesive (for example) or any other suitable adhesive. The durability sought to be achieved will determine the suitable approach for attachment.

In a further embodiment there is provided a means to indicate the volume of air that has passed through the vent, or alternatively the time that the vent assembly has been used. When a sufficient volume of air has passed through the vent assembly, or the assembly has been used for a sufficient time and may have become blocked, the indicator will signal that the vent assembly should be replaced.

For convenience, the thin air permeable membrane can be provided in an insert which is releasably attachable to the mask shell via a push-fit mechanism, as shown in Fig. 8. Preferably on at least the outer surface of the insert there is provided at least one cross-piece that protects the air permeable membrane from being damaged as it is located into the receiving orifice of the mask shell. This approach will allow for the easy placement, removal and replacement of a vent insert while retaining the other components of the mask. While the insert may be configured to take the form of any requisite shape preferably the insert has a circular circumferential shape defining a cylindrical insert which has a frictional fit within a corresponding circular orifice in the mask shell or gas inlet.

Formation of the vent through use of an insert configuration facilitates the selection and fitting of a vent to suit a user's requirements. For a fixed orifice vent, low flow occurs at low pressures and high flow occurs at high pressures. Therefore, a relatively large vent area may be adopted to facilitate achievement of the clinically desirable mask CO₂ washout rate. Should a higher treatment pressure be required then the previously selected vent may be exchanged for a vent that is more restrictive to flow. The more restrictive vent will allow achievement of the clinically desirable mask CO₂ washout rate while avoiding the intensity of noise and exhaust gas jetting that would occur had the previously selected low pressure vent been used with the higher treatment pressure.

Locating the vent in the mask shell results in an improvement in the minimization of CO₂ retention within the mask compared to locating the vent as an inline mask component.

In other further embodiments of the invention, another type of air permeable membrane may be used in the mask vents. Instead of the PTFE membrane described above, a vent of a mesh material may be used as an air permeable membrane. One suitable type of mesh material includes a PTFE mesh sold by Spectrum Laboratories of Rancho Dominguez, California, USA under the name Fluorocarbon SPECTRA/MESH®. In one preferred form, the SPECTRA/MESH® membrane has the following characteristics:
- Membrane material: Primarily if not 100% polytetrafluoroethylene mesh
- Mesh pore size: about 70-120 µm, preferably about 105 µm
- Open area: about 25-35%, preferably about 32%
- Thickness: about 120-180 µm, preferably about 155 µm

The PTFE mesh is preferably hydrophobic and may be used in the same manner as described above with respect to Figs. 2 and 6 for the ePTFE membrane.

PTFE mesh may also be used in a number of other vent configurations. For example, the PTFE mesh may be installed above an existing air vent or air restrictive element with essentially no gap between the PTFE mesh and the air vent. For example, PTFE mesh may be installed above an air vent such as the MIRAGE® air vent described above and in International Patent Application No. WO 98/34665 incorporated herein by reference in its entirety. In this configuration, the air vent would cause the majority of the pressure drop between the inside of the mask and the outside of the mask and the PTFE mesh would act to diffuse the air leaving the vent and reduce noise. Alternatively, PTFE mesh may be installed above an air vent with a small gap (e.g., 0.5 - 1 mm) between the vent and the PTFE mesh, such that the gap would help to diffuse the escaping air over a larger area of the mesh.

In any of the PTFE mesh configurations described above, one or several layers of PTFE mesh may be used. If multiple layers of PTFE mesh are used, the airflow restriction created by the mesh would be increased.

The PTFE mesh may be provided in the form of a disposable insert, similar to the inserts 82 of Figs. 6-8.

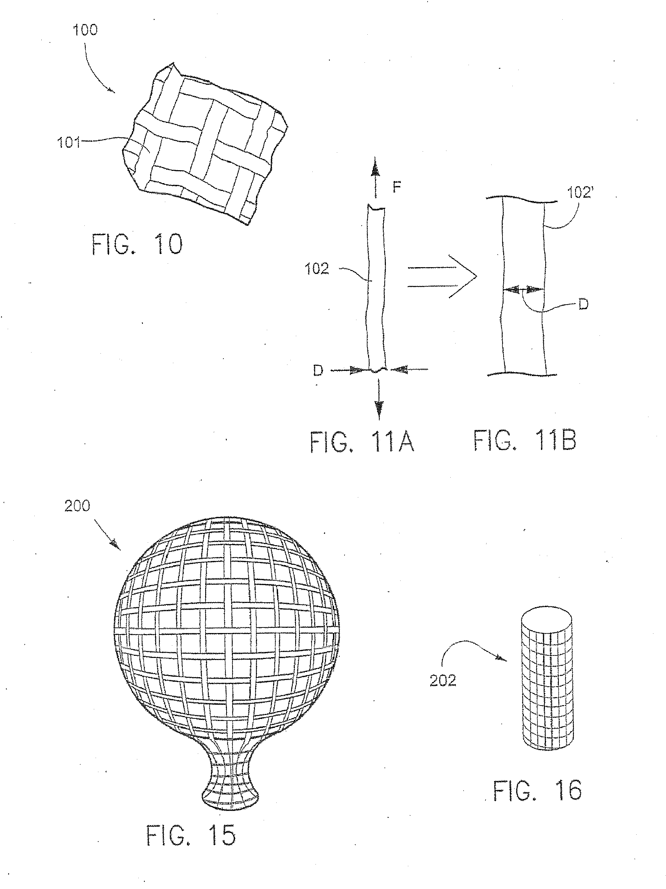

Figs. 10-16 illustrate a further embodiment of the invention in which the mesh material of the vent takes the form of a porous fabric, e.g., in the form of a loose weave 100. The weave 100 includes a plurality of fibers 102, at least some of which are made from an auxetic material meaning the fibers have a negative Poisson's Ratio (less than zero, although higher negative values are preferable) and/or a low Young's Modulus, e.g., in the range of 0.01 to 10 or higher. Preferably, the Young's Modulus (YM) is less than 10, and even more preferably, the YM is in the range of about 0.1. Examples include polymers such as polyimides (YM: 3-5), polyesters (YM: 1-5), nylon (YM: 2-4); polystyrene (YM: 3-3.4); polyethylenes (YM: 0.2-0.7); and/or rubbers (YM: 0.01-0.1). An auxetic material will thicken upon the application of a tensile force F to the fibers, such that the diameter d in Fig. 11A increases to diameter D in Fig. 11B. The following Internet links describe the theory and operation of auxetic materials:
http://www.azom.com/details.asp?ArticleID=167;
http://www.bolton.ac.uk/research/materials/pdf/c&i-review.pdf: and
http://www-moratti.ch.cam.ac.uk/projects/auxetics.html.

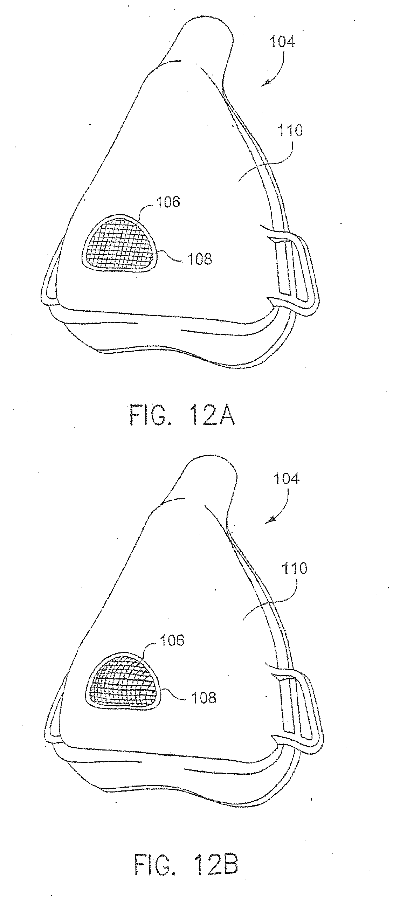

Figs. 12A and 12B show a mask 104 having a vent 106 in the form of a loose weave, as described above. The vent 106 may include a frame 108 that is inserted into an aperture of the mask shell 110. In Fig. 12A, the mask 104 is subject to the lower pressure range, in which case the weave is substantially planar, or slightly bulging. In Fig. 12B, when the mask is subject to the higher pressure range, the loose weave bulges out, in which case the auxetic fibers 102 assume the stretched condition and the diameter of the fibers increases, as explained above in relation to Figs. 11A and 11B. In this situation, the effective surface area of the loose weave is increased under higher pressures. However, the diameter of the fibers increases with stretching. Therefore, the spacing between the fibers (defining the open area) may remain constant, increase or decrease, depending on the desired open area between the fibers. Of course, the fibers may increase in diameter even if there is no bulging.

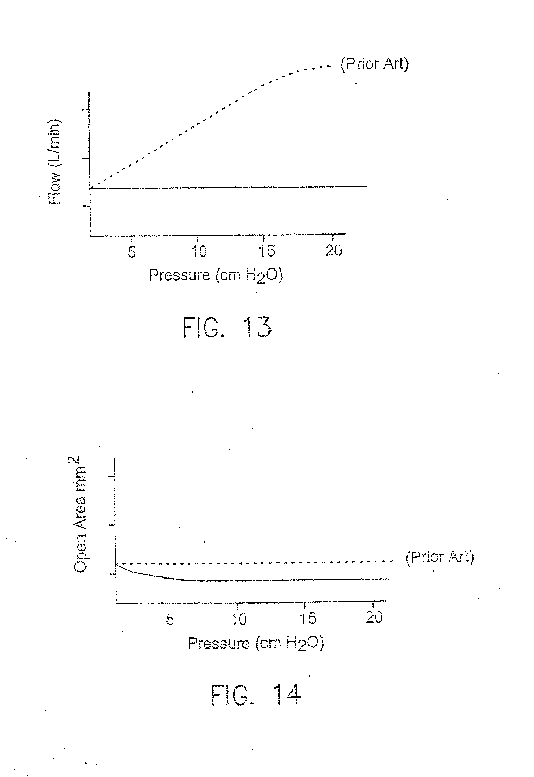

Fig. 13 plots Flow or flow rate (L/min) over Pressure (cmH₂O). The solid line represents a vent according to an embodiment using an auxetic material, e.g., a loose weave, with auxetic fibers. The flow remains relatively constant (in the ideal scenario), or rises very slowly to allow increased CO₂ washout, as pressure increases. By contrast, the flow of a prior art vent increases relatively more readily, e.g., in linear fashion, with pressure increases.

Fig. 14 plots the open area (mm²), i.e., the openings between the auxetic fibers, as pressure rises. Preferably, the open area is in the rang e of 60-90 mm², preferably about 75 mm². Ideally, the open area gradually decreases with pressure increases, to avoid unnecessarily high CO₂ washout. Of course, the open area could be designed to increase as well. In prior art masks having static or fixed holes, the open area remains constant.

The shape of the vent 106 may be symmetrical about at least one axis as shown in Figs. 12A-12B. However, the shape of the vent may be fully non-symmetrical, or it may be symmetrical about more than one axis, e.g., a round vent that bulges into the shape of a hemisphere or a portion of a hemisphere.

Figs. 15 and 16 show variations of the auxetic fiber vent. Fig. 15 shows a balloon-shaped vent 200, while Figs. 16 shows a cylindrical-shaped vent 202. These embodiments involve increased allowable expansion, i.e., stretch, which may increase or otherwise vary vent flow. In Figs. 15 and 16, the fibers in the areas of increased stretch have a larger diameter than the diameter of fibers in the areas that are stretched relatively less.

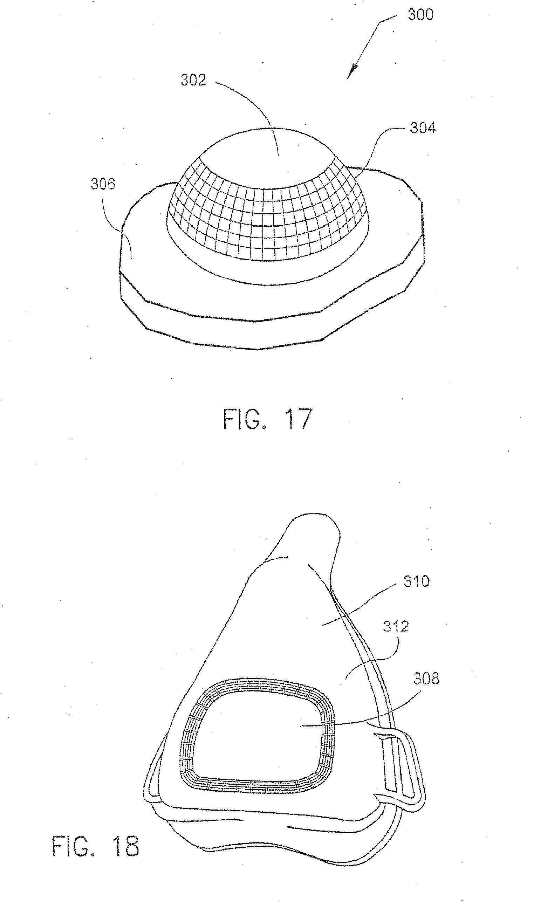

Fig. 17 shows yet another variation of a vent using an auxetic material. The vent 300 includes a first portion 302 made of non-porous material and a second portion 304 made of auxetic material. The first portion acts as a "sail" to thereby enhance the stretching force applied to the auxetic material. The sail force equals mask pressure times the area (F=PxA) of the first portion. Second portion 304 may be attached to a mask frame 306.

The sail portion 304 in Figure 17 may take other forms. For example, the sail portion 306 may be in the form of a polycarbonate cut out 308 that is attached to the remainder of the mask frame 310 using auxetic material 312, as shown in Fig. 18. The cutout 308 acts to vary the stretchy force applied to the auxetic material, is dependence of the prevailing mask pressure.

While the embodiments of Figs. 10-18 have been deserted in relation to auxetic materials in the form of a mesh or loose weave, it should be noted that the auxetic materials may take other forms. For example, the auxetic material could include a sheet of auxetic material having one or more slits and/or perforations.

Although the invention has been described with reference to specific examples, it is to be understood that these examples are merely illustrative of the application of the principles of the invention. Thus it is to be understood that numerous modifications may be made in the illustrative examples of the invention and other arrangements may be devised without departing from the spirit and scope of the invention.

## Claims

1. A respiratory mask comprising:
a cushion; and
a gas washout vent;
the gas washout vent comprising a mesh.

2. The mask of claim 1, the mesh taking the form of a porous fabric.

3. The mask of claims 1 or 2, wherein the mesh comprises a thermoplastic polymer, preferably PTFE.

4. The mask of any one of the preceding claims wherein the mesh is hydrophobic.

5. The mask of any one of the preceding claims, comprising a mask shell.

6. The mask of claim 5, comprising a breathable gas inlet that can conduct gas through the mask shell to a breathing cavity.

7. The mask of claims 5 or 6, wherein the cushion is positioned in proximity to the mask shell to aid in fitting the mask shell to a user's face.

8. The mask of claim 7, wherein the proximity is to an edge portion of the mask shell.

9. The mask according to any one of the preceding claims, the mesh has a pore size of about 70-120 µm, preferably about 105 µm.

10. The mask according to any one of the preceding claims, the mesh has a thickness of about 120µm to 180µm, preferably about 155µm.

11. The mask according to any one of the preceding claims, wherein the shape of the gas washout vent is symmetrical about at least one axis.
